# EUROPÄISCHE PATENTANMELDUNG

(11) **EP 1 489 201 A2**
(43) Veröffentlichungstag der Anmeldung: **22.12.2004**
(21) Anmeldenummer: 04013834.9
(22) Anmeldetag: 12.06.2004
(51) Int. Cl.: C25D 3/02

(54) **Propansulfonierte und 2-Hydroxy-propansulfonierte Alkylaminalkoxylate, ihre Herstellung und Verwendung als Hilfsmittel zur elektrolytischen Abscheidung von metallischen Schichten**

(30) Priorität: 18.06.2003 DE 10327374
(71) Anmelder: Raschig GmbH, 67061 Ludwigshafen (DE)
(72) Erfinder: Leifeld, Ferdinand, 67067 Ludwigshafen (DE); Urich, Rainer, 67117 Limburgerhof (DE); Ortmann, Isolde, 67123 Dannstadt (DE); Keller, Marcus, 67227 Frankenthal (DE)
(74) Vertreter: Wagner, Jutta, Dr.

(57) **Zusammenfassung**

Die vorliegende Erfindung betrifft die Verwendung von propansulfonierten oder 2-Hydroxy-propansulfonierten Alkylaminalkoxylaten bzw. deren Metallsalzen als Hilfsmittel zur elektrolytischen Abscheidung von metallischen Schichten, Hilfsmittel zur elektrolytischen Abscheidung von metallischen Schichten enthaltend Umsetzungsprodukte von Propansulton oder Chlorhydroxypropansulfonsäure mit Alkylaminalkoxylaten, sowie galvanische Bäder, die diese Hilfsmittel enthalten.

## Beschreibung

Die vorliegende Erfindung betrifft propansulfonierte oder 2-Hydroxy-propansulfonierte Alkylaminalkoxylate bzw. deren Metallsalze, ihre Herstellung, die Verwendung dieser Produkte als Hilfsmittel zur elektrolytischen Abscheidung von metallischen Schichten sowie galvanische Bäder, die diese als Hilfsmittel enthalten.

Es ist seit langem bekannt, daß sich bei der Abscheidung von metallischen Schichten aus galvanischen Bädern die Eigenschaften der Abscheidung, beispielsweise Glanz, Streuung und Korrosionsfestigkeit durch den Zusatz von organischen Hilfsmitteln verbessern lassen.

US-P 3,328,273 beschreibt die Abscheidung von Kupfer aus einem sauren, galvanischen Bad in Gegenwart von SPS, Polypropylenglykol und dem Farbstoff Janusgrün. In der DE-OS 25 41 897 wird die Kombination von SPS, ethoxilierten oder propoxilierten Polyethyleniminen, welche dann mit Benzylchlorid umgesetzt wurden, beschrieben. US-P 4,036,711 schlägt den Einsatz von quarternisierten Alkyl- und Arylaminen gemeinsam mit Sulphoalkylsulfiden und Poylethylenglykolen vor. In der DE-PS 20 39 831 wird die Verwendung von polymeren Phenazoniumverbindungen vorgestellt. Zur Verbesserung der Streuung und Metallverteilung wird in der DE-OS 196 43 091 der Einsatz von wasserlöslichen, mit Epichlorhydrin vernetzten Polyamiden beschrieben. Die DE-OS 101 00 954 schlägt den zusätzlichen Einsatz von Poylolefinen mit basischen, aromatischen Substituenten zur Verbesserung der Einebnung vor.

Das Verzinken aus schwach sauren Zinkbädern ist seit vielen Jahren bekannt. In der DE-OS 3613874 wird ein saures galvanisches Bad beschrieben, welches Leitsalze, aromatische oder heteroaromatische Ketone als Glanzbildner und sulfatierte sowie kernsulfonierte Alkylphenolalkoxylate als anionische Tenside zum Lösen des Glanzbildners enthält. Ferner kann das Bad noch nicht ionische Tenside, Polyethylenimine als Grundglanzbildner sowie Natriumbenzoat und Naphthalinsulfonsäure-Formaldehyd-Kondensationsprodukte zur Verbesserung der Streuung enthalten. Die DE-OS 3432956 schlägt die Verwendung von Sulfaten und Sulfoderivaten der β-Naphtholpolyglykolether in diesem Badtyp vor.

Schwach sauren Nickelbädern müssen bekannterweise bestimmte organische Substanzen zugefügt werden, um statt einer matten Abscheidung eine glänzende Nickelabscheidung zu erhalten. In der Patentschrift DE-PS 1004011 wird die Kombination von Leitsalzen, Netzmitteln, aromatischen Sulfimiden und Umsetzungsprodukten von aromatischen, heterozyklischen Stickstoffbasen mit Sultonen vorgestellt. DE-OS 2417787schlägt ein Nickelbad vor, in dem zu einer Basis von Nickelsalzen, Borsäure, acetylenisch ungesättigten Verbindungen, Pyridiniumsalzen und Sulfonsäureimiden noch ein Gemisch aus aromatischen Sulfonsäureimiden und Vinylsulfonsäure und/oder Allylsulfonsäure hinzugefügt wird. In der DE-OS 4211 140 wird zusätzlich der Einsatz von organisch substituierten Phosphoniumsalzen beschrieben.

Nicht umgesetzte Alkylaminalkoxylate werden in der Galvanotechnik in Zinn, Blei oder Zinn/Blei Legierungsbädern eingesetzt (US 4135991). Die PL 150671 beschreibt den Einsatz von ethoxilierten aliphatischen Aminen in Ätzbädern für Stahl und Stahlguß. Ethoxlierte Stearylamine können auch zur stromlosen Abscheidung von Kupferschichten verwendet werden (EP 132594).

Die bekannten galvanischen Bäder führen oft zu unbefriedigenden Ergebnissen in Bezug auf Glanz, Einebnung und Metallverteilung besonders in Bereichen mit niedrigen Stromdichten. Ein weiter Nachteil ist, daß die meisten Produkte nur in einem oder zwei Badtypen eingesetzt werden können und daß die kathodisch abgeschiedene Schicht häufig passiviert wird, was zu Haftungsproblemen bei einer anschließend abgeschiedenen Metallschicht führt.

Es bestand daher die Aufgabe Hilfsmittel zu finden, mit denen die Abscheidung metallischer Schichten verbessert werden kann.

Übrraschend wurde gefunden, dass durch eine Propansulfonierung oder 2-Hydroxypropansulfonierung von Alkylaminalkoxylaten Produkte entstehen, welche die oben genannte Aufgabe lösen.

Propansulfonierte und 2-Hydroxypropansulfonierte Alkylaminalkoxylate bzw. deren Metallsalze ergeben als Hilfsmittel bei der elektrolytischen Abscheidung metallischer Schichten in verschiedenen Badtypen nicht passivierte Oberflächen mit einer gleichzeitigen Verbesserung des Glanzes und der Einebnung besonders bei Abscheidung mit niedrigen Stromdichten.

Propansulfonierte bzw. Hydroxypropansulfonierte Alkylaminakoxylate sind bekannt. Die US 3,505,396 beschreibt die Ethoxylierung von Soja-, Kokos- und Talgaminen und deren anschließende Umsetzung mit Sulton, vorzugsweise Propansulton. Die Produkte sollen sich unter anderem als Tenside für Shampoos eignen. Die DE 21 59 834 beschreibt die Verwendung von Umsetzungsprodukten aus Amin, Alkylenoxid und Sulton oder Hydroxypropansulfonsäure als antistatischen Zusatz zu Polyacrylnitrilfasern. In DE 19 01 411 wird die Herstellung von Umsetzungsprodukten aus oxyalkylierten primären oder sekundärem Amin mit 3-Chlor-2-hydroxypropansulfonsäure beschrieben und der Einsatz als Zusatzmittel für Polyesterfärbeflotten offenbart. Keines der Dokumente enthält einen Hinweis auf eine Eignung der Umsetzungsprodukte für die elektrolytische Abscheidung metallischer Schichten.

Die erfindungsgemäßen eingesetzten Verbindungen entstehen bei der Reaktion von einem alkoxylierten Alkylamin mit 1,3-Propansulton oder 3-Chlor-2-hydroxypropansulfonsäuresalz. Zur Beschleunigung der Umsetzung kann die Reaktion in Gegenwart einer Base erfolgen. Als Base sind Alkali und Erdalkalioxide, - hydroxide, -alkoholate oder -carbonate bevorzugt. Besonders bevorzugt werden NaOH, KOH und Alkoholate von Kalium oder Natrium.

Die Alkylgruppe des Alkylamins hat vorzugsweise eine Kettenlänge von C₆ bis C₃₂, insbesondere von C₆ bis C₂₀. Bevorzugt werden primäre Amine eingesetzt. Ebenfalls einsetzbar sind alkoxylierte Dialkylamine. Die Alkylgruppen können geradkettig oder verzweigt, gesättigt oder ungesättigt und/oder substituiert sein. Als Alkylgruppen eignen sich insbesondere Hexyl, Ethylhexyl, Octyl, Decyl, Dodecyl, Tetradecyl, Hexadecal, Octadecyl und Oleyl.

Auch ein Gemisch von Aminen mit verschieden langen Alkylketten ist möglich. Bevorzugt sind hier Fettsäureamine, die aus den entsprechenden Fetten preiswert gewonnen werden können. Typische Verteilungen der Kettenlängen sind dabei:

| Amin | C₈ | C₁₀ | C₁₂ | C₁₄ | C₁₆ | C₁₈ | C₂₀ |
|---|---|---|---|---|---|---|---|
| Cocosfettamin | 4±2 | 6±2 | 52±4 | 18±3 | 10±3 | 10±3 | |
| Stearylamin | | | | 4 ± 3 | 30 ± 5 | 64 ± 4 | < 3 |
| Oleylamin | | | 1 | 4 | 12 | 83 | |
| Talgamin | | | < 1.5 | 4 ± 2 | 30 ± 7 | 65 ± 10 | < 2 |

Die Alkylaminalkoxylate werden in bekannter Weise durch Alkoxylierung der entsprechenden Alkylamine hergestellt. Die Alkylgruppe der Alkoxyreste sollte zwischen 2 und 6, vorzugsweise zwischen 2 und 4 C-Atome aufweisen. Sie kann geradkettig oder verzweigt sein. Besonders bevorzugt sind Ethoxy- oder Propoxyeinheiten. Die Anzahl n der Alkoxyeinheiten kann zwischen 3 und 50 Einheiten betragen, vorzugsweise zwischen 10 und 30 Einheiten. Vorzugsweise wird bei der Alkoxylierung von langkettigen Alkylaminen die Anzahl der Alkoxyeinheiten n geringer gehalten als bei der Alkoxylierung von kurzkettigen Aminen. Im Einzelfall können aber auch langkettige Amine mit großen Anzahlen n kombiniert werden.

Das Alkylaminalkoxylat kann mit einem oder zwei Mol 1,3-Propansulton bzw. 3-Chlor-2-hydroxypropansulfonsäure-Natriumsalz substituiert werden. Statt des Natriumsalzes der 3-Chlor-2-hydroxypropansulfonsäure sind auch andere Alkalioder Erdalkalialze sowie Ammoniumsalze brauchbar. Die Sulfopropylierung bzw. Hydroxysulfopropylierung erfolgt bevorzugt an den Hydroxylgruppen der Polyalkylenglykolkette, aber auch eine Quarternisierung der Aminfunktion ist möglich.

Die entstehenden Produkte der Propansultonreaktion sind Salze von N-((3-sulfopropyl)-polyalkoxy)-alkylamin oder Bis-(N-((sulfopropyl)-polyalkoxy))-alkylamin, auch eine Mischung beider Produkte ist möglich. Ebenfalls möglich ist die Bildung oder teilweise Bildung von N,N-Alkoxy-N-alkyl-N-sulfopropyl-betain. Betaine bilden sich bevorzugt, wenn die Umsetzung ohne Base erfolgt und die Produkte anschließend neutralisiert werden.

Bei der Umsetzung mit einem Salz der 3-Chlor-2-hydroxy-propansulfonsäure entstehen die Salze von N-((2-Hydroxy-3-sulfopropyl)-polyalkoxy)-alkylamin oder Bis-(N-((2-Hydroxy-3-sulfopropyl)-polyalkoxy))-alkylamin oder Mischungen beider Produkte. Ebenfalls möglich ist die Bildung oder teilweise Bildung von N,N-alkoxy-N-alkyl-N-(2-hydroxy-3-sulfopropyl)-betain.

Zur Durchführung der Reaktion wird das Alkylaminalkoxylat, bevorzugt in einem inerten Lösemittel, mit der Base zum Alkoholat umgesetzt. Zur Sulfopropylierung bzw. Hydroxysulfopropylierung wird anschließend Propansulton bzw. 3-Chlor-2-hydroxypropansulfonsäuresalz zudosiert. Nach Ablauf der Reaktion wird gegebenenfalls das Lösemittel abdestilliert. Zur Erhöhung der Fließfähigkeit kann dem Produkt anschließend noch Wasser zugesetzt werden. Auch eine Reaktion ohne Base mit einer anschließenden Neutralisation ist möglich. Als Lösemittel kommen alle gegenüber Alkoholaten inerten Lösemittel, wie zum Beispiel Toluol, Xylol oder DMF, in Frage. Ebenfalls möglich ist die Umsetzung direkt in Phase ohne Lösemittel. Die Reaktionstemperatur liegt vorzugsweise zwischen 30 und 150 °C, insbesondere zwischen 80 und 120 °C.

Die erfindungsgemäßen galvanischen Bäder enthalten neben den üblichen anorganischen und organischen Bestandteilen noch ein gemäß den oben beschriebenen Umsetzungen hergestelltes Hilfsmittel. Die Einsatzmengen der sulfopropylierten oder hydroxy-sulfopropylierten Alkylaminalkoxylate liegen in Bereichen von 0,001 bis 10 g/l Bad. Bevorzugte Anwendungsbäder sind solche zur galvanischen Abscheidung von Kupfer, Nickel, Zink und seinen Legierungen sowie Zinn und seinen Legierungen aus sauren Bädern. Andere galvanisch abscheidbare Metalle, insbesondere Silber, Gold und Platin, können voraussichtlich ebenfalls vorteilhaft abgeschieden werden.

Das saure Kupferbad enthält bevorzugt neben dem beschriebenen Produkt noch weitere organische Zusätze. Vorteilhaft ist eine Kombination mit mindestens einer durch Sulfopropylierung wasserlöslich gemachten Schwefelverbindungn. In der folgenden Tabelle 1 sind hierzu einige Beispiele mit den bevorzugten Einsatzmengen angegeben:

**Tabelle 1:**

| Schwefelverbindungen als Zusatz in galvanischen Bädern | | |
|---|---|---|
| Schwefelverbindung | Chemischer Charakter | Konzentration im Bad in g/l |
| SPS | Bis-(3-Natrium-sulfopropyl)-disulfid | 0,0005 - 0,5 |
| MPS | 3-Mercapto-1-propansulfonsäure-Natriumsalz | 0,001 - 1 |
| ZPS | 3-(2-Benzthiazolyl-2-mercapto)-propansulfonsäure-Natriumsalz | 0,002 - 0,5 |
| DPS | N,N-Dimethyl-dithiocarbaminsäure-(3-sulfopropyl)-ester-Natriumsalz | 0,001 - 0,1 |
| OPX | O-Ethyl-dithiokohlensäure-(3-sulfopropyl)-ester-Kaliumsalz | 0,001 - 0,5 |
| UPS | S-Isothiuronium-3-propansulfonat | 0,0005 - 0,1 |

Zusätzlich können im Bad noch Netzmittel, Polyether, Polyolefine, mit Epichlorhydrin vernetzte Polyamide und weitere im sauren Kupferbad übliche Zusätze enthalten sein. Als Netzmittel können beta-Naphthol-alkoxylate wie beispielsweise Ralufon NO 14 (Raschig GmbH, Ludwigshafen, DE) mit 2,5 Mol Propylenoxid und 14 Mol Ethylenoxid in Mengen von 0,001 bis 1 g/l verwendet werden, als vernetztes Polyamid Raluplate 2887 (Raschig GmbH, Ludwigshafen, DE) in Mengen von 0,001 bis 0,5 g/l, sowie als Polyolefin Raluplate LEV 170 (Raschig GmbH, Ludwigshafen, DE) in Mengen von 0,001 bis 1 g/l.

Die anorganischen Bestandteile des sauren Kupferbades sind vorzugsweise Kupfersulfat, Schwefelsäure und Chloride, es können aber andere Kupfersalze und statt Schwefelsäure auch andere Säuren wie beispielsweise Fluoroborsäure oder Methansulfonsäure verwendet werden. Meistens enthält das Bad zwischen zwischen 50 und 250 g/l Kupfersulfat (CuSO₄ * 5H₂O), zwischen 25 und 250 g/l Schwefelsäure und zwischen 0,01 und 0,5 g/l Chloridionen.

Saure Nickelbäder können neben dem beschriebenen Umsetzungsprodukt noch Sulfonsäureimide wie Saccharin und Pyridiniumverbindungen wie PPS (Pyridiniumpropylsulfobetain), PPSOH (2-Hydroxy-3-sulfopropyl-pyridinium-betain) oder SPV (1-(3-Sulfopropyl)-2-vinyl-pyridinium-betain) enthalten. Möglich ist auch eine Kombination mit acetylenischen Verbindungen wie z.B. Propargylalkohol und Butindiol sowie deren Derivaten. Das Bad kann weiterhin ungesättigte Verbindungen wie Allylsulfonate oder Vinylsulfonate und auch Netzmittel enthalten. Die verwendeten wäßrig sauren Nickelelektrolyt Bäder enthalten ein oder meist mehrere Nickelsalze, z.B. Nickelsulfat oder Nickelchlorid und eine oder mehrere anorganische Säuren, vorzugsweise Borsäure und Schwefelsäure. Üblicherweise enthält das Bad zwischen 200 bis 350 g/l NiSO₄*7H₂O, 30 bis 150 g/l NiCl₂*6H₂O sowie 30 bis 50 g/l H₃BO₃. Der pH-Wert des Elektrolyten liegt zwischen 3 und 6 vorzugsweise zwischen 4 und 5.

Das Zinkbad kann neben der erfindungsgemäßen Verbindung noch aromatische Ketone oder Aldehyde wie Benzalaceton oder o-Chlorbenzaldehyd, organische Säuren wie Benzoesäure sowie nicht ionische und anionische Netzmittel enthalten. Üblicherweise wird als Badlösung für das Zinkbad eine wässrige Lösung von 30 - 120 g/l Zinkchlorid (ZnCl₂) und 60 - 200 g/l Kaliumchlorid verwendet. Es können auch andere Zinksalze wir Zinksulfat, Zinkacetat oder Zinkfluoroborat verwendet werden. Statt Kaliumchlorid kann teilweise oder auch ganz Ammoniumchlorid, Ammoniumsulfat oder ein anderes Leitsalz verwendet werden. Weitere Zusätze können Borsäure oder andere Puffergemische sein. Der pH Bereich des Bades liegt zwischen 3,0 - 6,0, vorzugsweise zwischen 4,5 - 5,5 und die Temperatur zwischen 10°C und 60 °C, vorzugsweise zwischen 20°C und 50 °C.

Die Erfindung wird anhand der folgenden Beispiele näher erläutert ohne jedoch darauf beschränkt zu sein.

### Beispiel 1

Zu 0,5 Mol eines propoxilierten Stearylamins (n = 15) werden bei Raumtemperatur 500 ml Xylol und 1 Mol Natrium-methylat hinzugefügt. Das Gemisch wird erhitzt und zuerst das entstehende Methanol abdestilliert. Bei einer Reaktionstemperatur von ca. 100°C wird 1 Mol Propansulton zudosiert und zur Nachreaktion 2 Stunden bei 100 °C gerührt. Das Xylol wird im Vakuum abdestilliert. Zur Erhöhung der Fließfähigkeit werden 300 g Wasser hinzugefügt. Das Endprodukt ist eine braune, honigviskose Flüssigkeit.

| | | |
|---|---|---|
| Gehalt | 65 - 75 % | gemäß HClO₄-Titration |
| Wassergehalt | 35 - 25 % | Karl-Fischer Titration |
| pH - Wert | 8-11 | 10 %-ig in Wasser |

### Beispiel 2:

Zu 0,5 Mol eines ethoxilierten Cocosfettamins (n = 20) werden 500 ml Xylol und 0,5 mol KOH Pulver hinzugefügt. Das Gemisch wird auf Rückfluß erhitzt und das entstehende Reaktionswasser azeotrop abdestilliert. Bei einer Reaktionstemperatur von ca. 100 °C wird 0,5 Mol Propansulton hinzugefügt. Nach etwa 2 Stunden Nachreaktion wird das Xylol abdestilliert.

| | | |
|---|---|---|
| Gehalt | 95-100 % | gemäß HCIO4-Titration |
| Wassergehalt | 0 - 5 % | Karl Fischer Titration |
| PH-Wert | 8 - 11 | 10 %-ig in Wasser |

### Beispiel 3:

### Zusammensetzung des Grundbades:

| | |
|---|---|
| 210 g/l | Kupfersulfat*5H₂O |
| 75 g/l | Schwefelsäure |
| 1 ml/l | Salzsäure 10 %ig |

### Organische Badadditive:

| | |
|---|---|
| 0,05 g/l | Polyethylenglykol (Mg. ca. 9000 g/mol) |
| 0,02 g/l | SPS |
| 0,02 g/l | DPS |
| 0,07 g/l | Raluplate LEV 170 (Polymeres Vinylpyridin; Mg: ca. 15000g/mol) |

Ein bei 25 °C, bei Luftbewegung und bei 2 A in einer Hullzelle mit einer Kupferschicht galvanisch beschichtetes Messingblech zeigt einen leicht verschleierten Glanz und eine Einebnung bis zu einem Stromdichtebereich von 1,5 A/dm². Dem Elektrolyten werden 0,10 g/l des Produktes aus Beispiel 1 hinzugefügt. Das nachfolgend beschichtete Hullzellblech ist im gesamten Stromdichtebereich hoch glänzend und zeigt eine Einebnung bis zu einem Stromdichtebereich von ca. 0,2 A/dm². Die ohne Zwischenaktivierung auf der Kupferschicht aufgebrachte Nickelschicht hat eine sehr gute Haftung und zeigt keinerlei Risse beim Biegetest.

### Beispiel 4:

### Zusammensetzung des Grundbades:

| | |
|---|---|
| 330 g/l | Nickelsulfat*7H₂O |
| 70 g/l | Nickelchlorid*6H₂O |
| 50 g/l | Borsäure |

### Organische Badadditive:

| | |
|---|---|
| 2,00 g/l | Saccharin-Natriumsalz |
| 0,200 g/l | Pyridiniumpropylsulfobetain |
| 0,5 g/l | Natrium-Allylsulfonat |
| 0,01 g/l | Propargyl-3-sulfopropylether-Natriumsalz |

Die bei 54 °C, Luftbewegung und 2 A in einer Hullzellle auf einem Messingblech galvanisch abgeschiedene Nickelschicht zeigt eine Einebnung bis ca. 1,5 A/dm2. Durch die Zugabe von 0,01 g/l des Produktes aus Beispiel 1 kann der Einebnungsbereich bis 0,1 A/dm2 in die niedrige Stromdichte geschoben werden. Eine nachfolgend aufgebrachte Chromschicht zeigt eine hervorragende Haftung.

### Beispiel 5:

### Zusammensetzung des Grundbades:

| | |
|---|---|
| 80 g/l | Zinkchlorid |
| 200 g/l | Kaliumchlorid |
| 25 g/l | Borsäure |

### Organische Badadditive:

| | |
|---|---|
| 2,0 g/l | Sulfopropyliertes β-Naphtolalkoxylat-Kaliumsalz |
| 2,0 g/l | Ethylhexanol-ethoxylat |
| 0,12 g/l | Benzalaceton |
| 2,0 g/l | Natrium-Benzoat |

Mit dem beschriebenen Elektrolyten wird bei 25 °C und 1,5 A in einer Hullzelle auf ein Stahlblech galvanisch eine Zinkschicht abgeschieden. Zur Elektrolytbewegung wird Luft in die Zelle eingeblasen. Die Zinkschicht zeigt einen verschleierten Glanz in den niedrigen Stromdichten und nur eine geringe Einebnung. Der Zusatz von 0,1 g/l des Produktes aus Beispiel 2 bewirkt einen brillianten Glanz und eine verbesserte Einebnung im gesamten Stromdichtebereich.

### Beispiel 6:

Zu 0,5 Mol eines ethoxylierten 2-Ethylhexylamins (n = 5) werden bei Zimmertemperatur 500 ml Toluol und 0,5 Mol 50%ige Natronlauge-Lösung in Wasser hinzugefügt. Das Gemisch wird auf Rückfluß erhitzt und das Wasser aus der Natronlauge und das entstehende Reaktionswasser werden azeotrop abdestilliert. Bei einer Reaktionstemperatur von ca. 70 °C wird 1,3-Propansulton zudosiert und zur Nachreaktion 2 Stunden bei 80 °C gerührt. Toluol wird im Vakuum abdestilliert und zur Erniedrigung der Viskosität werden 50 g Wasser hinzugefügt. Das Endprodukt ist eine viskose braune Flüssigkeit.

| | | |
|---|---|---|
| Gehalt | 85-90 % | gemäß HCIO₄-Titration |
| Wassergehalt | 8 - 11 % | Karl Fischer Titration pH: 8 - 11 |

### Beispiel 7

### Zusammensetzung des Grundbades:

| | |
|---|---|
| 330 g/l | Nickelsulfat*7H₂O |
| 70 g/l | Nickelchlorid*6H₂O |
| 50 g/l | Borsäure |

### Organische Badadditive:

| | |
|---|---|
| 2,00 g/l | Saccharin-Natriumsalz |
| 0,20 g/l | 1-(2-Hydroxy-3-sulfopropyl)-pyridinium betain |
| 0,03 g/l | Butinyl-glycerin ether |

Die bei 54 °C, Luftbewegung und 2 A in einer Hullzelle auf einem Messingblech galvanisch abgeschiedene Nickelschicht zeigt einen gleichmäßigen, aber nicht brillianten Glanz und nur eine geringe Einebnung bis zu einem Stromdichtebereich von ca. 2 A/dm². Durch die Zugabe von 0,03 g/l des Produktes aus Beispiel 7 können Glanz und Einebnung verbessert und bereits in einem Stromdichtebereich von 0,1 A/dm² erhalten werden.

## Patentansprüche

1. Verwendung von Propansulfonierten oder 2-Hydroxypropansulfonierten Alkylaminalkoxylaten als Hilfsmittel bei der Abscheidung metallischer Schichten, wobei die Alkylgruppe des Alkylamins eine Kettenlänge von 6 bis 32 C-Atomen hat und das Amin mit 3 bis 50 Alkoxyeinheiten alkoxyliert ist.

2. Verwendung gemäß Anspruch 1, **dadurch gekennzeichnet, dass** die Alkylgruppe gesättigt und/oder unverzweigt ist.

3. Verwendung gemäß einem der Ansprüche 1 oder 2, **dadurch gekennzeichnet, dass** Alkylgruppen verschiedener Kohlenstoffatomanzahl vorhanden sind.

4. Verwendung gemäß einem der Ansprüche 1 bis 3, **dadurch gekennzeichnet, dass** 10 bis 30 Alkoxyeinheiten vorhanden sind.

5. Hilfsmittel zur elektrolytischen Abscheidung metallischer Schichten enthaltend propansulfonierte oder 2-Hydroxypropansulfonierte Alkylaminalkoxylate, wobei die Alkylgruppe des Alkylamins eine Kettenlänge von mit 6 bis 32 C-Atomen hat und das Amin mit 3 bis 50 Alkoxyeinheiten alkoxyliert ist.

6. Hilfsmittel gemäß Anspruch 5, **dadurch gekennzeichnet, dass** propansulfonierte Alkylaminalkoxylate der Formel 1 enthalten sind 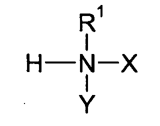 wobei
R¹ für eine Alkylgruppe mit 6 bis 32 Kohlenstoffatomen und
X für die Gruppe 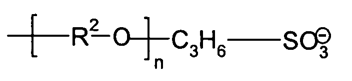 steht, wobei
R² für CH₂-CH₂ oder CH₂-CH(CH₃) steht,
n eine ganze Zahl von 3 bis 50 bedeutet,
und Y für H, R¹ oder X steht.

7. Hilfsmittel gemäß Anspruch 5, **dadurch gekennzeichnet, dass** 2-Hydroxypropansulfonierte Alkylaminalkoxylate der Formel II enthalten sind 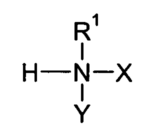 wobei
R¹ für eine Alkylgruppe mit 6 bis 32 Kohlenstoffatomen und
X für die Gruppe 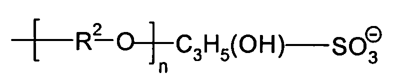 steht,
wobei R² für CH₂-CH₂ oder CH₂-CH(CH₃) steht und
n eine ganze Zahl von 3 bis 50 bedeutet,
und Y für H oder X steht.

8. Hilfsmittel gemäß Anspruch 5, **dadurch gekennzeichnet, dass** Alkylaminalkoxylate der Formel III enthalten sind 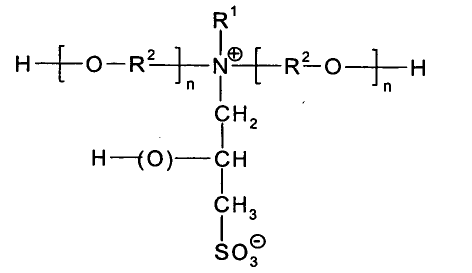 wobei
R¹ für eine Alkylgruppe mit 6 bis 32 Kohlenstoffatomen
R² für CH₂-CH₂ oder CH₂-CH(CH₃) steht und
n eine ganze Zahl von 3 bis 50 bedeutet.

9. Galvanische Bäder enthaltend Hilfsmittel zur elektrolytischen Abscheidung metallischer Schichten gemäß mindestens einem der Ansprüche 5 bis 8.

10. Galvanische Bäder gemäß Anspruch 9, **dadurch gekennzeichnet, dass** von 0,001 bis 10 g/l Bad der sulfopropylierten oder hydroxy-sulfopropylierten Alkylaminalkoxylate enthalten sind.

11. Galvanische Bäder gemäß Anspruch 9 oder 10, **dadurch gekennzeichnet, dass** sie zur galvanischen Abscheidung von Kupfer, Nickel, Zink und seinen Legierungen sowie Zinn und seinen Legierungen aus sauren Bädern bestimmt sind.
